# EUROPÄISCHE PATENTANMELDUNG

(11) **EP 0 606 937 A1**
(43) Veröffentlichungstag der Anmeldung: **20.07.1994**
(21) Anmeldenummer: 94200020.9
(22) Anmeldetag: 06.01.1994
(51) Int. Cl.: A61B 6/00

(54) **Röntgengerät mit einem um eine horizontale Schwenkachse schwenkbaren Geräteteil**

(30) Priorität: 14.01.1993 DE 4300796
(71) Anmelder: Philips Patentverwaltung GmbH, 22335 Hamburg (DE); Philips Electronics N.V., 5621 BA Eindhoven (NL)
(72) Erfinder: Hove, Ulrich, Dr.-Ing., D-20097 Hamburg (DE)
(74) Vertreter: Hartmann, Heinrich, Dipl.-Ing.

(57) **Zusammenfassung**

Die Erfindung betrifft ein Röntgengerät mit einem um eine horizontale Schwenkachse (3) außerhalb seines Schwerpunktes schwenkbaren Geräteteil (9,2) und mit einer Federanordnung (7) zur Kompensation des von dem Geräteteil ausgeübten Drehmoments, die an einem Koppelglied (8) angreift, das bei einer Schwenkung des Geräteteils eine Kreisbahn (100) um die Schwenkachse ausführt. Dabei wird mit geringem Aufwand ein guter Gewichtsausgleich dadurch erreicht, daß der Mittelpunkt (11) der Kreisbahn in einer die Schwenkachse enthaltenden senkrechten Ebene außerhalb der Schwenkachse liegt, und daß das Koppelglied in einer mit dem Geräteteil verbundenen und auf die Schwenkachse zu verlaufenden Führungseinrichtung (9,90) geführt ist.

## Beschreibung

Die Erfindung betrifft ein Röntgengerät mit einem um eine horizontale Schwenkachse außerhalb seines Schwerpunktes schwenkbaren Geräteteil und mit einer Federanordnung zur Kompensation des von dem Geräteteil ausgeübten Drehmoments, die an einem Koppelglied angreift, das bei einer Schwenkung des Geräteteils eine Kreisbahn um die Schwenkachse ausführt. Wenn das Drehmoment kompensiert und somit ein Gewichtsausgleich erreicht ist, kann ein Benutzer auch ein schweres Geräteteil mit geringem Kraftaufwand schwenken.

Ein solches Röntgengerät ist aus der EP-A1-0498255 bekannt. Bei dem bekannten Röntgengerät ist das Geräteteil - eine Röntgenaufnahme-Einheit eines Mammographiegerätes - fest mit einem kurbelförmigen Koppelglied verbunden, das bei einer Schwenkung des Geräteteils eine Kreisbahn um die Schwenkachse beschreibt. Das Koppelglied wirkt über eine Laufrolle auf ein Teil ein, das eine horizontale Lauffläche für die Laufrolle aufweist und vertikal verschiebbar ist. Auf dieses Teil wirkt ein Gewichtsausgleichssystem ein. Wenn die von diesem System gelieferte Ausgleichskraft konstant ist, kann bei geeigneter Auslegung das von dem Geräteteil bezüglich der Schwenkachse ausgeübte Drehmoment kompensiert werden. Eine konstante Ausgleichskraft könnte von einem Ausgleichsgewicht geliefert werden.

Wenn das Gewichtsausgleichssystem aber mit einer Federanordnung arbeitet, verändert diese bei einer Schwenkung des Geräteteils ihre Länge und damit auch die vom Gewichtsausgleichssystem erzeugte Kraft. Das Drehmoment kann dann allenfalls noch in einer oder zwei Schwenkstellungen vollständig kompensiert werden. Es kommt hinzu, daß bei dem bekannten Röntgengerät das Gewichtsausgleichssystem nicht nur das beim Schwenken vom Geräteteil ausgeübte Drehmoment kompensieren, sondern auch das Gewicht dieses Geräteteils ausgleichen soll. Die vom Gewichtsausgleichssystem gelieferte Kraft muß daher dem Gewicht des Geräteteils entsprechen oder - falls das System mit dem Teil über einen Flaschenzug in Verbindung steht, in einem ganzzahligen Verhältnis zu der Kraft stehen. In jedem dieser Fälle entspricht die Längenänderung der Federanordnung der Verschiebung des Geräteteils in vertikaler Richtung. Sie kann daher relativ groß sein und entsprechend große Änderungen der vom Gewichtsausgleichssystem gelieferten Federkraft zur Folge haben, so daß das zur Kompensation erzeugte Drehmoment relativ stark von dem vom Geräteteil ausgeübten Moment abweichen kann.

Aufgabe der vorliegenden Erfindung ist es, ein Röntgengerät der eingangs genannten Art so auszugestalten, daß das beim Schwenken des Geräteteils sich ergebende Drehmoment einfach und relativ genau kompensiert werden kann.

Diese Aufgabe wird dadurch gelöst, daß der Mittelpunkt der Kreisbahn in einer die Schwenkachse enthaltenden senkrechten Ebene außerhalb der Schwenkachse liegt, und daß das Koppelglied in einer mit dem Geräteteil verbundenen und auf die Schwenkachse zu verlaufenden Führungseinrichtung geführt ist. Bei der Erfindung fällt der Mittelpunkt der Kreisbahn also nicht mit der Schwenkachse zusammen, sondern liegt in einer die Schwenkachse enthaltenden senkrechten Ebene. Dadurch ergibt sich auch über einen großen Schwenkwinkelbereich ein guter Gewichtsausgleich. Da sich dabei das Koppelglied auf einer bezüglich der Schwenkachse exzentrischen Bahn bewegt, stellt die Führungseinrichtung sicher, daß das Koppelglied sich stets auf der den Schwerpunkt des Geräteteils mit der Schwenkachse verbindenden Wirkungsgerade befindet.

Um das Koppelglied auf einer zur Schwenkachse exzentrischen Kreisbahn zu bewegen, könnte ein mit dem Koppelglied verbundener Kurbelarm vorgesehen sein, der um eine zweite Achse schwenkbar ist, die parallel zur Schwenkachse verläuft. In diesem Fall wäre aber der Schwenkbereich des Geräteteils eingeschränkt. Solche Einschränkungen werden bei einer Ausgestaltung der Erfindung dadurch vermieden, daß das Koppelglied mit einem Träger verbunden ist, der in einem Lagerungsring gelagert ist, dessen Mittelachse mit der Schwenkachse nicht zusammenfällt.

Eine andere Weiterbildung der Erfindung sieht vor, daß das Koppelglied und der Schwerpunkt des Geräteteils sich auf verschiedenen Seiten der Schwenkachse befinden und daß der Mittelpunkt der Kreisbahn unterhalb der Schwenkachse liegt. Wenn sich das Koppelglied und das Geräteteil sich auf derselben Seite der Schwenkachse befänden, müßte der Mittelpunkt der Kreisbahn oberhalb der Schwenkachse liegen.

In Ausgestaltung der Erfindung ist vorgesehen, daß Verstellmittel zum Verstellen des Abstandes zwischen dem Mittelpunkt der Kreisbahn und der Schwenkachse vorgesehen sind, die so gestaltet sind, daß auch bei einer Verstellung der Mittelpunkt und die Schwenkachse eine senkrechte Ebene definieren. Die Differenz zwischen dem von dem Geräteteil bezüglich der Schwenkachse erzeugten Drehmoment und dem mittels der Federanordnung zur Kompensation erzeugten Drehmoments hängt außer von der Schwenkstellung auch von der Federcharakteristik, insbesondere der Federkonstanten ab. Bei Verwendung von Schraubenfedern können sich aber Fertigungsstreuungen der Federkonstanten in der Größe von 15 % ergeben. Durch Verstellen des Abstandes zwischen dem Mittelpunkt der Kreisbahn und der Schwenkachse läßt sich der Gewichtsausgleich unterschiedlichen Federkonstanten anpassen.

Eine bevorzugte Weiterbildung der Erfindung sieht vor, daß das Geräteteil die Röntgenaufnahme-Einheit eines Mammographiegerätes ist, die einen Röntgenstrahler und eine Objektlagerungseinheit umfaßt, daß der Röntgenstrahler wahlweise gemeinsam mit der Objektlagerungseinheit oder unabhängig von ihr um die Schwenkachse schwenkbar ist und daß die Schwenkachse durch den Schwerpunkt der Objektlagerungseinheit verläuft. Der Gewichtsausgleich ist in diesem Fall unabhängig davon, ob das Röntgengerät gemeinsam mit der Objektlagerungseinheit geschwenkt wird oder unabhängig davon (letzteres ist für Biopsie-Untersuchungen wichtig).

Die Erfindung wird nachstehend anhand der Zeichnungen näher erläutert. Es zeigen:
- Fig. 1a: ein erfindungsgemäßes Röntgenuntersuchungsgerät in schematischer Darstellung einer Seitenansicht.
- Fig. 1b: das gleiche Gerät in einer Rückansicht.
- Fig. 2a: zeigt die Differenz zwischen dem Drehmoment des Geräteteils und dem kompensierenden Moment als Funktion der Winkelstellung bei optimaler Einstellung.
- Fig. 2b: die Differenz als Funktion des Schwenkwinkels für verschiedene Federkonstanten bei nicht optimaler Einstellung.

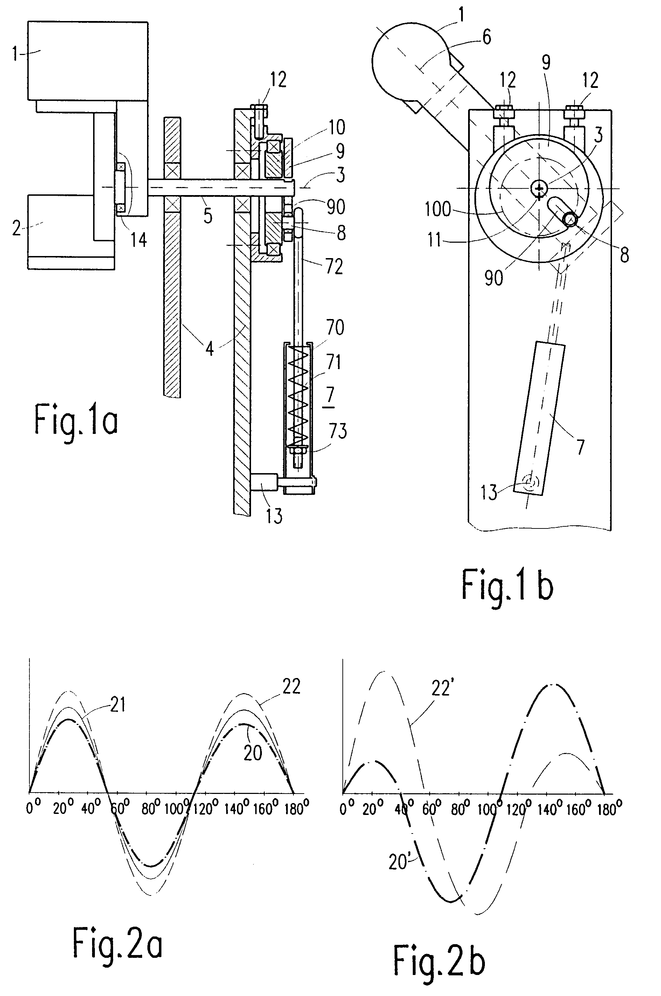

In Fig. 1a und 1b ist ein Teil eines Röntgenuntersuchungsgeräts für die Mammographie in einer Seiten- bzw. einer Rückansicht dargestellt. Das Geräteteil, das um eine horizontale Achse geschwenkt werden soll, wird in diesem Fall durch eine Röntgnaufnahme-Einheit gebildet, die einen Röntgenstrahler 1 und eine Objektlagerungseinheit 2 umfaßt. Diese Einheit ist - um einer horizontale Achse 3 schwenkbar - in einem Stativ 4 gelagert, von dem nur die Stützwände teilweise dargestellt sind. Die Röntgenaufnahme-Einheit 1, 2 ist dabei mit einer Welle 5 verbunden, deren Mittelachse die Schwenkachse 3 ist und die in dem Stativ gelagert ist.

Ebenso wie bei dem Gerät nach der EP-A1 498 255 liegt die Schwenkachse etwa im Zentrum eines Untersuchungsobjektes. Da der Röntgenstrahler 1 aber wesentlich schwerer ist als die Objektlagerungseinheit 2, verläuft die Schwenkachse 3 nicht durch den Schwerpunkt; dieser befindet sich vielmehr in der Nähe des Röntgenstrahlers 1. Sobald die Verbindungsebene, die die Schwenkachse 3 mit dem Schwerpunkt verbindet und die in Fig. 1b mit 6 bezeichnet ist, nicht vertikal verläuft, übt die Röntgenaufnahme-Einheit 1, 2 ein Drehmoment bezüglich der Achse 3 aus.

Zum Gewichtsausgleich, d.h. zur Kompensation dieses Drehmoments, ist eine Federanordnung 7 vorgesehen, die um den zur Welle 5 parallelen Lagerzapfen 13 schwenkbar ist, der sich unterhalb der Welle 5 befindet und mit dieser eine senkrechte Ebene definiert; dadurch sind die Eigenschaften des Gewichtsausgleichs unabhängig davon, ob die Einheit 1, 2 nach links oder nach rechts geschwenkt wird. Die Federanordnung 7 umfaßt ein Federgehäuse 70, in dem sich eine Schraubenfeder 71 befindet; bei einer Schraubenfeder hängt die Federkraft praktisch linear mit dem Federweg zusammen. Die Schraubenfeder 71 umschließt eine Stange 72, die durch eine Öffnung in der oberen Stirnfläche des Gehäuses 70 geführt ist und an ihrem unteren Ende ein Gewinde aufweist. Die Schraubenfeder 71 wird mit ihrem oberen Ende gegen die Stirnfläche gedrückt, wobei mit einer Stellmutter 73 die Vorspannung der Schraubenfeder 71 einstellbar ist. Obwohl dadurch die Schraubenfeder zusammengedrückt wird, wird auf die Stange 72 eine Zugkraft ausgeübt.

Diese Zugkraft wird von der Stange 72 auf ein Koppelglied 8 in Form eines Zapfens übertragen, das sich in der Ebene 6 befindet. Das Koppelglied 8 ist in einem senkrecht auf die Schwenkachse zu verlaufenden Langloch 90 in einem mit der Welle 5 verbundenen Flansch 9 geführt, so daß es sich in der Ebene 6 entweder auf die Schwenkachse 3 zu oder von ihr wegbewegen kann. Der Zapfen 8 ist mit einem Ring 10 verbunden, der die Welle 5 umschließt und dessen Mittelachse parallel zur Schwenkachse 3 verläuft. Bei einer Schwenkung des Strahlers 1 verschiebt sich daher der Zapfen 8 innerhalb des Langlochs 90 und bewegt sich dabei gleichzeitig auf einer zur Schwenkachse 3 senkrechten Kreisbahn 100. Der Mittelpunkt 11 dieser Kreisbahn und die Schwenkachse 3 liegen in einer vertikalen Ebene, wobei im Ausführungsbeispiel der Mittelpunkt unterhalb der Schwenkachse 3 liegt.

Das Lager, das den Ring 10 mit dem Kurbelzapfen 8 trägt, kann in vertikaler Richtung mittels der Stellschrauben 12 verstellt werden, so daß die Lage des Kreises 100 bzw. seines Mittelpunktes 11 in bezug auf die Schwenkachse 3 in einer vertikalen Ebene verstellt werden kann.

Die von der Federanordnung 7 auf den Zapfen 8 ausgeübte Zugkraft erzeugt ein Drehmoment um die Schwenkachse 3, das dem von der Röntgenaufnahme-Einheit 1, 2 ausgeübten Drehmoment entgegengerichtet ist. Durch geeignete Bemessung der Federkraft ist daher eine exakte Kompensation der Drehmomente bzw. ein Gewichtsausgleich möglich, allerdings nicht für beliebige Neigungen der Röntgenaufnahme-Einheit 1, 2.

Das von der Röntgenaufnahme-Einheit 1, 2 erzeugte Drehmoment ist für die Schwenkwinkel 0° und 180° (der Schwenkwinkel ist der Winkel zwischen der Senkrechten und der Ebene 6) Null. Das gleiche gilt für das in diesen Stellungen von der Federanordnung ausgeübte Drehmoment. Zwischen 0° und 180° ändert sich das von der Einheit 1, 2 erzeugte Drehmoment proportional zum Sinus des Schwenkwinkels. Das von der Federanordnung erzeugte Drehmoment hat nur annähernd einen solchen sinusförmigen Verlauf, so daß eine exakte Kompensation nicht für alle Schwenkwinkel möglich ist.

Wenn der Mittelpunkt 11 der Kreisbahn 100, auf der der Zapfen bewegbar ist, mit der Schwenkachse zusammenfallen würde - wie bei der Anordnung nach der EP-A1 498 255 - dann ließe sich die Federanordnung so einstellen, daß noch bei einem weiteren Schwenkwinkel - z.B. bei 90° - eine exakte Kompensation der Drehmomente erreicht wird. Bei kleineren Schwenkwinkeln überwiegt das Drehmoment der Einheit 1, 2, weil die Feder 71 dabei weniger zusammengedrückt wird und eine kleinere Zugkraft erzeugt. Bei größeren Schwenkwinkeln hingegen überwiegt das von der Feder erzeugte Drehmoment, weil die Feder 71 stärker zusammengedrückt wird und damit eine höhere Zugkraft auf den Zapfen 8 ausübt. Die Abweichungen zwischen den Drehmomenten können dabei relativ groß sein, so daß ein Benutzer die Einheit 1, 2 nur mit einigem Kraftaufwand in eine gewünschte Position bringen kann.

Wenn stattdessen, wie aus Fig. 1 ersichtlich, der Mittelpunkt 11 unterhalb der Schwenkachse 3 liegt, wird mit zunehmendem Schwenkwinkel (bei ansteigender Federkraft) der Abstand zwischen dem Zapfen 8 und der Schwenkachse 3 kleiner. Dies gestattet es, den Gewichtsausgleich so zu dimensionieren, daß eine exakte Kompensation (außer bei 0° und 180°) noch bei einem ersten und bei einem zweiten Schwenkwinkel auftritt (das gleiche gilt wegen der Symmetrie der Anordnung auch für eine Schwenkung der Einheit 1, 2 von der vertikalen Ausgangslage in die entgegengesetzte Richtung). Dabei überwiegt bei kleinen Schwenkwinkeln das Drehmoment der Einheit 1, 2. In einem mittleren Schwenkwinkelbereich dominiert das Drehmoment der Federanordnung, und für darüber liegende Schwenkwinkel bis unterhalb von 180° dominiert wieder das Drehmoment der Einheit 1, 2. Die Abweichungen von der exakten Kompensation der Drehmomente können dabei um ein Mehrfaches geringer sein als bei einer Anordnung, bei der der Mittelpunkt 11 und die Schwenkachse 3 zusammenfallen. Die optimale Dimensionierung ergibt sich dabei, wenn die maximalen Abweichungen der Drehmomente voneinander in allen drei Schwenkwinkelbereichen dem Betrage nach gleich groß sind.

Dies ist in Fig. 2a dargestellt, die qualitativ den Verlauf der Differenz der Drehmomente von Röntgenaufnahme-Einheit 1, 2 und Federanordnung als Funktion des Schwenkwinkels (zwischen 0 und 180°) darstellt. Die ausgezogene Kurve 21 wurde für eine bestimmte Federkonstante erhalten, nachdem der Abstand zwischen dem Mittelpunkt 11 und der Schwenkachse 3 mittels der Stellschrauben 12 und die Federvorspannung mittels der Stellschraube 73 optimiert wurde.

Diese Optimierung und ihre Wirkung soll an dem folgenden Zahlenbeispiel verdeutlicht werden. Bei einem Abstand des Schwerpunktes der Einheit 1, 2 von der Schwenkachse 3 von 500 mm betrug deren Gesamtmasse 40 kg. Der Abstand zwischen dem Lagerzapfen 13 und der Schwenkachse 3 betrug 600 mm und der Radius der Kreisbahn war 60 mm. Bei einer Federkonstanten von 14 N/mm ergab sich für den Schwenkwinkelbereich von 0 bis 180° ein Optimum für eine Federvorspannung (das ist die minimale Federkraft, die sich ergibt, wenn der Kurbelzapfen den untersten Punkt auf der Kreisbahn einnimmt) von 2362 N und einen Abstand zwischen Schwenkachse 3 und Mittelpunkt 11 von 9,5 mm. Die maximale Abweichung zwischen den Drehmomenten lag in diesem Fall bei rund 3000 Nmm. Bei dem angegebenen Schwerpunktabstand bedeutet dies, daß die 40 kg schwere Einheit 1, 2 mit einem Kraftaufwand von maximal 6 N geschwenkt werden kann. Dies ist für die klinische Praxis zumutbar.

In Fig. 2a ist zusätzlich noch eine gestrichelte Kurve 22 und eine strichpunktierte Kurve 20 dargestellt. Diese Kurven stellen das Optimum dar, wenn eine Feder mit einer ca. 6,5 % größeren (Kurve 22) bzw. kleineren (Kurve 20) Federkonstanten verwendet wird. Diese Abweichung der Federkonstanten liegen im Rahmen der Fertigungsstreuungen bei den handelsüblichen Federn. Um das Optimum zu erreichen, mußten die Federvorspannung und der Abstand zwischen Schwenkachse und Mittelpunkt der geänderten Federkonstanten angepaßt werden. Bei einer größeren Federkonstanten (Kurve 22) ist eine geringere Federvorspannung, aber ein größerer Abstand zwischen Schwenkachse und Mittelpunkt erforderlich; bei der kleineren Federkonstante (Kurve 20) ist es genau umgekehrt.

Hätte man bei einer geänderten Federkonstanten hingegen nur die Federvorspannung geändert und den Abstand zwischen Schwenkachse 3 und Mittelpunkt 11 konstant gelassen (9,5 mm), dann würden sich günstigstenfalls die in Fig. 2b dargestellten Kurven 20 und 22' ergeben, denen die gleichen Federkonstanten zugrundeliegen wie den Kurven 20 und 22 in Fig. 2a.

Man erkennt, daß für ein Optimum stets die Federkonstante, die Federvorspannung und der erwähnte Abstand aufeinander abgestimmt werden müssen. Wenn einer dieser drei Parameter der Optimierung geändert wird, vergrößern sich die Abweichungen.

Wird beispielsweise der Abstand zwischen Schwenkachse und Mittelpunkt kleiner gewählt als für das Optimum erforderlich, dann werden die Abweichungen der Drehmomente bei kleinen Schwenkwinkeln größer und bei großen Schwenkwinkeln kleiner. Im Vergleich zu dem optimierten Fall wird nämlich der Abstand zwischen dem Zapfen 8 und Schwenkachse 3 bei kleinen Schwenkwinkeln kleiner und bei großen Schwenkwinkeln größer, so daß auch das von der Federanordnung erzeugte Drehmoment kleiner bzw. größer wird. - Ist hingegen der Abstand zwischen Mittelpunkt 11 und Schwenkachse 3 größer als für das Optimum erforderlich, dann werden - bei sonst unveränderten Parametern - die Abweichungen bei kleinen Schwenkwinkeln kleiner und bei großen Schwenkwinkeln größer. Wenn man darüberhinaus in dem letztgenannten Fall die Federvorspannung etwas vergrößert, wird erreicht, daß sich die Abweichungen bei kleinen und großen Schwenkwinkeln vergrößern und den im mittleren Schwenkwinkelbereich verkleinern. Dadurch ergeben sich bis zu mittleren Schwenkwinkeln von z.B 110° kleinere Abweichungen als sie bei den für den gesamten Schwenkwinkelbereich von 0 bis 180° optimierten Kurven möglich sind. Dies ist dann von Vorteil, wenn die Röntgenaufnahme-Einheit auf der Vertikalen nicht um 180°, sondern z.B. nur um 90° geschwenkt werden soll.

Die Änderung der Federvorspannung bei sonst unveränderten Parametern der Optimierung beeinflußt ebenfalls die Abweichungen zwischen den Drehmomenten. Wenn die Federvorspannung vergrößert wird, vergrößert sich - bei sonst unveränderten Parametern - die Federkraft und damit auch das von der Feder erzeugte Moment, so daß sich die Kurven 20 bis 22 der Fig. 2a nach unten verschieben, d.h. die positiven Abweichungen zwischen den Drehmomenten werden kleiner und die negativen dem Betrage nach größer. - Bei einer Verringerung der Federkraft verschieben sich die Kennlinien der Fig. 2a hingegen nach oben.

Bei dem in Fig. 1 dargestellten System liegt die Schwenkachse zwischen dem Schwerpunkt der Einheit 1, 2 und dem Zapfen 8. Der Gewichtsausgleich wird hierbei dadurch erreicht, daß auf den Zapfen 8 ein Zug ausgeübt wird. Es wäre jedoch auch möglich, den Gewichtsausgleich zu realisieren, indem im Bereich zwischen der Schwenkachse 3 und dem Schwerpunkt der Einheit 1,2 ein Druck ausgeübt wird. Zu diesem Zweck müßte der Lagerring gegenüber der in Fig. 1b dargestellten Position um 180° verdreht sein, und die Federanordnung 7 müßte als Druckfeder wirken. Ein Optimum entsprechend Fig. 2 könnte in diesem Fall aber nur dann erreicht werden, wenn sich der Mittelpunkt 11 oberhalb der Schwenkachse 3 befinden würde.

Bei dem Ausführungsbeispiel nach Fig. 1 ist der Lagerzapfen aus Symmetriegründen in einer die Schwenkachse 3 enthaltenden vertikalen Ebene angeordnet - und zwar unterhalb der Schwenkachse. Ein Gewichtsausgleich wäre jedoch auch mit einer in der genannten Ebene oberhalb der Schwenkachse 3 angelenkten Lagerzapfen 13 möglich; allerdings müßte die Federanordnung 7 dann auf den Kurbelzapfen einen Druck ausüben. Auch in diesem Fall müßte der Mittelpunkt 11 unterhalb der Schwenkachse 3 liegen.

Für die üblichen mammographischen Röntgenuntersuchungen müssen sowohl der Röntgenstrahler 1 als auch die Objektlagerungseinheit 2 um die Schwenkachse 3 geschwenkt werden. Bei Biopsien jedoch, bei denen die Position einer Biopsienadel unter Röntgenkontrolle aus zwei verschiedenen Perspektiven aufgenommen wird, soll nur der Röntgenstrahler 1 bewegt werden, während die Objektlagerungseinheit 2 eine definierte Lage - z.B. unterhalb der Schwenkachse 3 - einhalten muß. Deshalb ist die Objektlagerungseinheit 2 mittels eines Lagers 14 an der Welle 5 drehbar gelagert, während der Röntgenstrahler 1 fest mit der Welle 5 verbunden ist. Durch nicht näher dargestellt Koppeleinrichtungen kann die Objektlagerungseinheit 2 starr mit dem Röntgenstrahler verbunden werden, so daß sie gemeinsam mit diesem um die Schwenkachse 3 geschwenkt werden kann. Damit in beiden Fällen mit einer einzigen Federanordnung der Gewichtsausgleich erfolgen kann, muß dann die Objektlagerungseinheit 2 in ihrem Schwerpunkt gelagert sein.

Bei der zuvor erläuterten Anordnung liegt die Kreisbahn, die der Zapfen 8 beschreibt, in einer Ebene, die zur Schwenkachse 3 senkrecht steht. Prinzipiell ist es aber auch möglich, daß die Ebene, in der die Kreisbahn liegt, die Schwenkachse unter einem von 90° abweichenden Winkel schneidet, wenn die Normale auf die Ebene horizontal verläuft. Dadurch verkürzt sich nämlich bei einem Schwenkwinkel im Bereich um 90° der Abstand zwischen dem Zapfen und der Schwenkachse, so daß das von der Feder erzeugte Drehmoment in diesem Winkelbereich abnimmt; dies hat zur Folge, daß die negativen Abweichungen in diesem Schwenkwinkelbereich kleiner werden. Die könnte zu einer Verbesserung des Gewichtsausgleichs führen; allerdings wäre dabei die Führung des Zapfens und dessen Verbindung mit der Feder wesentlich schwieriger.

## Patentansprüche

1. Röntgengerät mit einem um eine horizontale Schwenkachse (3) außerhalb seines Schwerpunktes schwenkbaren Geräteteil (1, 2) und mit einer Federanordnung (7) zur Kompensation des von dem Geräteteil ausgeübten Drehmoments, die an einem Koppelglied (8) angreift, das bei einer Schwenkung des Geräteteils eine Kreisbahn (100) um die Schwenkachse (3) ausführt,
dadurch gekennzeichnet, daß der Mittelpunkt (11) der Kreisbahn (100) in einer die Schwenkachse (3) enthaltenden senkrechten Ebene außerhalb der Schwenkachse (3) liegt, und daß das Koppelglied (8) in einer mit dem Geräteteil verbundenen und auf die Schwenkachse zu verlaufenden Führungseinrichtung (9, 90) geführt ist.

2. Röntgengerät nach Anspruch 1,
dadurch gekennzeichnet, daß das Koppelglied (8)mit einem Träger (10) verbunden ist, der in einem Lagerungsring drehbar gelagert ist, dessen Mittelachse (11) mit der Schwenkachse (3) nicht zusammenfällt.

3. Röntgengerät nach einem der vorhergehenden Ansprüche,
dadurch gekennzeichnet, daß das Koppelglied (8) und der Schwerpunkt des Geräteteils (1, 2) sich auf verschiedenen Seiten der Schwenkachse (3) befinden und daß der Mittelpunkt (11) der Kreisbahn (100) unterhalb der Schwenkachse (3) liegt.

4. Röntgengerät nach Anspruch 1,
dadurch gekennzeichnet, daß Verstellmittel (12) zum Verstellen des Abstandes zwischen dem Mittelpunkt (11) der Kreisbahn (100) und der Schwenkachse (3) vorgesehen sind, die so gestaltet sind, daß auch bei einer Verstellung der Mittelpunkt (11) und die Schwenkachse (3) eine senkrechte Ebene definieren.

5. Röntgengerät nach einem der vorhergehenden Ansprüche,
dadurch gekennzeichnet, daß die Federanordnung (7) eine Schraubenfeder (71) enthält.

6. Röntgengerät nach einem der vorhergehenden Ansprüche,
dadurch gekennzeichnet, daß Verstellmittel (73) zur Verstellung der Vorspannung der Federanordnung (7) vorgesehen sind.

7. Röntgengerät nach einem der vorhergehenden Ansprüche,
dadurch gekennzeichnet, daß das Geräteteil die Röntgenaufnahme-Einheit (1,2) eines Mammographiegerätes ist, die einen Röntgenstrahler (1) und eine Objektlagerungseinheit (2) umfaßt, daß der Röntgenstrahler (1) wahlweise gemeinsam mit der Objektlagerungseinheit (2) oder unabhängig von ihr um die Schwenkachse (3) schwenkbar ist und daß die Schwenkachse durch den Schwerpunkt der Objektlagerungseinheit verläuft.
